# EUROPEAN PATENT APPLICATION

(11) **EP 4 227 410 A1**
(43) Date of publication of application: **16.08.2023**
(21) Application number: 21877734.0
(22) Date of filing: 08.10.2021
(51) Int. Cl.: C12N 15/09, C12N 9/90, C12P 19/02

(54) **METHOD FOR MANUFACTURING KETOSE USING NOVEL KETOSE-3-EPIMERASE**

(30) Priority: 09.10.2020 JP 2020171513
(71) Applicant: Amano Enzyme Inc., Nagoya-shi Aichi 460-8630 (JP)
(72) Inventor: SAKAI Kiyota, Kakamigahara-shi, Gifu 509-0109 (JP); YACHI Hiroyuki, Kakamigahara-shi, Gifu 509-0109 (JP); YUKI Kensuke, Kakamigahara-shi, Gifu 509-0109 (JP); KAMON Masahiro, Kitanagoya-shi, Aichi 481-8533 (JP)
(74) Representative: Witthoff Jaekel Steinecke Patentanwälte PartG mbB
(86) International application number: PCT/JP2021/037275
(87) International publication number: WO 2022/075435

(57) **Abstract**

This invention provides a method for producing allulose using an allulose-producing enzyme having high pH stability in a low-pH region. The method comprises a step of allowing a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose to react with fructose under acidic conditions in which pH is less than 6.

## Description

### Technical Field

The present invention relates to a method for producing ketose using novel ketose-3-epimerase having high pH stability in a low-pH region.

### Background Art

In recent years, the demand for the rare sugar, such as allulose, that has a low calorie content and functionality is increasing because of enhanced health consciousness.

It is possible to synthesize allulose from D-fructose with the aid of ketose-3-epimerases including D-allulose-3-epimerase, D-tagatose-3-epimerase, and L-ribulose-3-epimerase, and allulose-producing enzymes derived from *Pseudomonas cichorii* and *Arthrobacter globiformis* have been known (Patent Documents 1 and 2).

When producing the rare sugar, such as allulose, the reactivity in the acidic region is preferable in order to prevent browning. However, existing enzymes had low reactivity in the acidic region and thus were not suitable for the treatment in the acidic region. While the *Dorea* sp.-derived allulose-producing enzymes are known to exert relatively high reactivity in the acidic region, such reactivity is not sufficient (Non-Patent Document 1).

### Prior Art Documents

### Patent Documents

Patent Document 1: JP H03-266996 A (1991)
Patent Document 2: WO 2014/109254

### Non-Patent Documents

Non-Patent Document 1: Zhang, 2015, J. Mol. Catal., B. Enzym.

### Summary of the Invention

### Objects to Be Attained by the Invention

The present invention provides a method for producing ketose using novel ketose-3-epimerase, which is acidophilic and acid-resistant.

### Means for Attaining the Objects

The present inventors have conducted concentrated studies in order to search for allulose-producing enzymes having high reactivity in the acidic region, so as to prevent allulose from browning when producing allulose, which is a type of ketose and referred to as the rare sugar. The present inventors searched for microorganisms producing allulose-producing enzymes that would exhibit high reactivity in the acidic region from the strain library of Amano Enzyme Inc. As a result, they discovered useful enzymes (in terms of the conversion rate and the reactivity in the acidic region) in *Asaia krungthepensis,* thereby completing the present invention.

Specifically, the present invention is as described below.
[1] A method for producing allulose comprising a step of allowing a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose to react with fructose under acidic conditions in which pH is less than 6.
[2] The method according to [1], wherein the acidic conditions in which pH is less than 6 are the conditions in which pH is less than 5.
[3] The method according to [1] or [2], wherein the polypeptide is allowed to react with fructose at 50°C to 70°C.
[4] The method according to any of [1] to [3], wherein the polypeptide is derived from *Asaia krungthepensis.*
[5] The method according to any of [1] to [4], wherein a microbial cell comprising a gene encoding a polypeptide capable of producing allulose in a cell is brought into contact with fructose.
[6] An enzyme preparation comprising, as an active ingredient, a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose.
[7] An enzyme preparation comprising a microorganism comprising a gene encoding a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose.
[8] The enzyme preparation according to [6] or [7], wherein the polypeptide is derived from *Asaia krungthepensis.*

This description includes part or all of the content as disclosed in the description and/or drawings of Japanese Patent Application No. 2020-171513, which is a priority document of the present application.

### Effects of the Invention

The allulose-producing enzyme derived from *A. krungthepensis* (AkDAE) according to the present invention has the reactivity in the acidic region where pH is less than 5. The allulose-producing enzyme according to present invention also has the reactivity in a high-temperature range of 50°C or higher. When allulose is produced with the aid of AkDAE, allulose can be prevented from browning, and allulose can be produced with a high conversion rate.

### Brief Description of the Drawings

Fig. 1 shows the results of the allulose production test using *A. krungthepensis* fragmentation solution.
Fig. 2 shows the optimal pH of AkDAE.
Fig. 3 shows pH stability of AkDAE.
Fig. 4 shows the optimal temperature of AkDAE.
Fig. 5 shows heat stability of AkDAE.
Fig. 6 shows substrate specificity of AkDAE.
Fig. 7 shows the rare sugar conversion rate of AkDAE.
Fig. 8 shows the results of comparison of the reactivity of AkDAE and that of known allulose-producing enzymes under neutral pH and acidic pH conditions.
Fig. 9 shows the results of the allulose production test using *E. coli* cells.

### Embodiments of the Invention

Hereafter, the present invention is described in detail.

The present invention relates to a method for producing allulose using A. *krungthepensis*-derived allulose-producing enzymes (AkDAE).

Allulose is a monosaccharide having the structure represented by Formula (I) and is classified as a ketose. Allulose is also referred to as "psicose". Since the amount thereof existing in nature is small, it is referred to as the rare sugar.

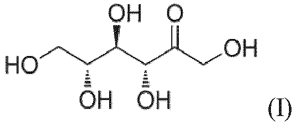

Allulose has about 70% sweetness of sucrose and 10% calories of sucrose. Thus, allulose has been reported to have functions of, for example, suppressing an increase in postprandial glucose levels and preventing obesity.

### 1. Allulose-producing enzyme derived from A. krungthepensis

The allulose-producing enzyme according to the present invention is derived from *Asaia krungthepensis*, the genus *Asaia*, the family *Acetobacteraceae*, and such enzyme can be isolated from *A. krungthepensis.* The allulose-producing enzyme derived from *A. krungthepensis* according to the present invention is D-allulose-3-epimerase (abbreviated as "AkDAE") and it is a type of ketose-3-epimerase.

SEQ ID NO: 5 shows the nucleotide sequence of DNA encoding AkDAE. SEQ ID NO: 6 shows the amino acid sequence of the enzyme according to the present invention.

The enzyme according to the present invention may comprise an amino acid sequence derived from SEQ ID NO: 6 by mutation, such as deletion, substitution, or addition of at least 1, and preferably 1 or several amino acids, provided that a protein comprising such amino acid sequence has activity of producing allulose from a fructose substrate.

For example, at least 1, and preferably 1 or several (e.g., 1 to 10, more preferably 1 to 5, and particularly preferably 1 or 2) amino acids may be deleted from the amino acid sequence shown in SEQ ID NO: 6, at least 1, and preferably 1 or several (e.g., 1 to 9, more preferably 1 to 5, and particularly preferably 1 or 2) amino acids may be added to the amino acid sequence shown in SEQ ID NO: 6, or at least 1, and preferably 1 or several (e.g., 1 to 9, more preferably 1 to 5, and particularly preferably 1 or 2) amino acids in the amino acid sequence shown in SEQ ID NO: 6 may be substituted with other amino acids.

An example of an amino acid sequence derived from the amino acid sequence shown in SEQ ID NO: 6 by deletion, substitution, or addition of 1 or several amino acids is an amino acid sequence having at least 85%, preferably at least 90%, more preferably at least 95%, more preferably at least 97%, more preferably at least 98%, and particularly preferably at least 99% sequence identity to the amino acid sequence shown in SEQ ID NO: 6 when calculated with the use of, for example, BLAST (Basic Local Alignment Search Tool at the National Center for Biological Information (NCBI)) (using, for example, default parameters).

A protein comprising an amino acid sequence derived from the amino acid sequence shown in SEQ ID NO: 6 by deletion, substitution, or addition of 1 or several amino acids is substantially identical to the protein comprising the amino acid sequence shown in SEQ ID NO: 6.

Also, DNA capable of hybridizing, under the stringent conditions described below, to DNA comprising a sequence complementary to the DNA comprising the nucleotide sequence shown in SEQ ID NO: 5 and encoding a protein having activity of producing allulose from a fructose substrate is within the scope of DNA encoding AkDAE according to the present invention. Under the stringent conditions, specifically, DNA can be identified by performing hybridization with the use of a filter comprising DNA immobilized thereon in the presence of 0.7 to 1.0 M NaCl at 68°C, followed by washing using a 0.1× to 2× SSC solution (a 1× SSC solution comprises 150 mM NaCl and 15 mM sodium citrate) at 68°C. Alternatively, DNA may be able to form a hybrid when DNA is transferred and immobilized on a nitrocellulose membrane by Southern blotting and then allowed to react in a hybridization buffer (50% formamide, 4× SSC, 50 mM HEPES (pH 7.0), 10× Denhardt's solution, 100 µg/ml salmon sperm DNA) at 42°C overnight.

In addition, DNA having at least 85%, preferably at least 90%, more preferably at least 95%, more preferably at least 97%, more preferably at least 98%, and particularly preferably at least 99% sequence identity to DNA comprising the nucleotide sequence shown in SEQ ID NO: 5 when calculated with the use of, for example, BLAST (Basic Local Alignment Search Tool at the National Center for Biological Information (NCBI)) (using, for example, default parameters) and encoding a protein having activity of producing allulose from a fructose substrate is within the scope of DNA encoding AkDAE according to the present invention.

The enzymatic chemical properties of AkDAE are as described below.

### (1) Activity

AkDAE produces allulose from a fructose substrate. The conversion rate of fructose into allulose by AkDAE is 25% or higher, and preferably 27% or higher, when 30 to 60 (w/v)% of the fructose substrate is used.

### (2) Optimal pH

While the optimal pH of AkDAE is 6 to 7, at a pH of 5 to 10, AkDAE has 80% or more activity of the activity at the optimal pH. Such optimal pH is measured with the use of a 50 mM acetate buffer (pH 4.0 to 6.0), a 50 mM phosphate buffer (pH 6.0 to 7.0), a 50 mM Tris-HCl buffer (pH 7.0 to 9.0), and a 50 mM glycine-NaOH buffer (pH 9.0 to 11.0).

### (3) pH stability

AkDAE is stable at pH of 4 to 10, and, at pH of 3 to 10, 60% or more activity remains after the treatment at 4°C for 24 hours. When the activity at a pH of 7.0 is designated 100%, the residual activity at a pH of 3.0 is 60% or more, and preferably 65% or more. When the activity at a pH of 7.0 is designated 100%, the residual activity at a pH of 4.0 is 90% or more, and preferably 95% or more.

### (4) Optimal temperature

While the optimal temperature of AkDAE is 55°C to 65°C, at 45°C to 75°C, AkDAE has 80% or more activity of the activity at the optimal temperature. The optimal temperature is measured using a 50 mM Tris-HCl buffer (pH 7.0).

### (5) Heat stability

AkDAE is a heat-resistant allulose-producing enzyme. When AkDAE is treated at 50°C or higher for 1 hour, the residual activity is about 100% at 50°C and 60°C, and the residual activity is 65% or more, and preferably 70% at 70°C.

### (6) Substrate specificity

AkDAE produces D-allulose from a D-fructose substrate, and it produces D-sorbose from a D-tagatose substrate. The epimerase activity of AkDAE is reversible. Thus, AkDAE produces D-fructose from a D-allulose substrate, and it produces D-tagatose from a D-sorbose substrate. Concerning substrate specificity, the reactivity to D-allulose is the highest, and the reactivity to D-fructose is 60%.

### (7) Rare sugar conversion rate

The rare sugar conversion rate is the proportion of a reactant to a product when an enzymatic reaction reaches a plateau. The rare sugar conversion rate of AkDAE is, for example, D-Frc:D-All is 70.9:29.1, and D-Tag:D-Sor is 69.4:30.6.

### (8) Molecular weight

The molecular weight of AkDAE is 32.2 kDa.

### 2. Method for producing AkDAE

AkDAE can be produced by culturing *A. krungthepensis,* collecting from the culture solution or cells, and purifying.

Culture conditions and culture methods are not particularly limited, provided that the enzyme of interest is produced. Specifically, methods and conditions suitable for culture of microorganisms to be used can be adequately selected, so as to produce the enzyme according to the present invention. A method of liquid culture or solid culture may be employed, with liquid culture being preferable. Conditions of liquid culture are described herein.

A medium is not particularly limited, provided that the microorganisms to be used can grow therein. For example, a medium supplemented with a carbon source, such as glucose, sucrose, gentiobiose, soluble starch, glycerin, dextrin, molasses, or organic acid, a nitrogen source, such as ammonium sulfate, ammonium carbonate, ammonium phosphate, ammonium acetate, peptone, yeast extract, corn steep liquor, casein hydrolysate, bran, or meat extract, and inorganic salt, such as potassium salt, magnesium salt, sodium salt, phosphate, manganese salt, iron salt, or zinc salt can be used. For the purpose of growth promotion of transformants to be used, vitamins, amino acids, and the like may be added to the medium. pH of the medium is adjusted to, for example, about 3 to 8, and preferably about 4 to 7, and culture is performed generally at about 20°C to 40°C, and preferably about 25°C to 35°C, for 1 to 10 days, and preferably about 3 to 6 days, under aerobic conditions. A method of shake culture, a method of aerobic submerged culture using ajar fermenter, or other methods can be employed.

After the culture performed under the conditions described above, the target enzyme is collected from a culture solution or cells. When the enzyme is to be collected from a culture solution, for example, a culture supernatant is first filtered or centrifuged to remove insoluble matters. Thereafter, concentration through an ultrafiltration membrane, salting out such as ammonium sulfate precipitation, dialysis, and various chromatography techniques, such as ionexchange resin, are performed in adequate combination to perform isolation and purification. Thus, the target enzyme can be obtained. When the target enzyme is to be collected from cells, in contrast, cells are fragmented by, for example, pressurization or ultrasound treatment, and the target protein can be obtained by separation and purification in the same manner as described above. Alternatively, cells may be collected from a culture solution in advance by filtration, centrifugation, or other means and may then be subjected to the procedure (fragmentation, separation, and purification of cells).

Also, recombinant AkDAE can be produced with the use of recombinant DNA containing the AkDAE gene. For example, an adequate host cell (e.g., an *E. coli* cell) may be transformed with DNA encoding the target enzyme (a specific example is DNA comprising the nucleotide sequence shown in SEQ ID NO: 5), and a protein expressed in the transformant may be collected. Thus, recombinant AkDAE can be produced. The collected protein is adequately prepared in accordance with the purpose of use. When the target enzyme is obtained in the form of a recombinant protein as described above, various modifications can be performed. For example, DNA encoding the target enzyme and another adequate DNA may be inserted into the same vector, and a recombinant protein may be produced with the use of the vector. Thus, the target enzyme comprising a recombinant protein comprising an arbitrary peptide or protein ligated thereto can be obtained. Also, addition of a sugar chain and/or lipid or a modification causing N- or C-terminal processing may be performed. As a result of the modification as described above, extraction and purification of a recombinant protein can be simplified or biological functions can be added to a recombinant protein.

### 3. Enzyme preparation

The enzyme according to the present invention can be provided in the form of an enzyme preparation. The enzyme preparation according to the present invention comprises, as an active ingredient, the enzyme according to the present invention. The enzyme preparation according to the present invention can be provided in the form of a microbial cell containing the enzyme according to the present invention in a cell. The enzyme preparation comprises a microbial cell containing a gene encoding the enzyme according to the present invention in a cell. Microorganisms are not particularly limited, provided that the microorganisms can express and accumulate the enzyme according to the present invention therein. Examples thereof include, *E*. *coli*, bacteria of the genus *Bacillus*, acetic acid bacteria, filamentous fungi, *Actinomycetes*, and yeast, with *E. coli* being preferable. Such microbial cells can be produced in the form of transformants by introduction of the enzyme gene into the microorganism. A transformant can be produced in accordance with a conventional technique. The transformant may be brought into contact with and allowed to react with fructose. The transformant expresses an enzyme in the cell, and the enzyme acts on fructose. After the microorganism acts on fructose and allulose is produced, the microorganism may be subj ected to ultrasonic fragmentation or other treatment, and microbial fragments may then be removed by centrifugation or other means. The enzyme preparation according to the present invention may comprise, in addition to an active ingredient (i.e., the enzyme of the present invention), an excipient, a buffer, a suspending agent, a stabilizer, a preservative, an antiseptic agent, physiological saline, and the like. Examples of excipients that can be used include starch, dextrin, maltose, trehalose, lactose, D-glucose, sorbitol, D-mannitol, saccharose, and glycerol. Examples of buffers that can be used include phosphate, citrate, and acetate buffers. Examples of stabilizers that can be used include propylene glycol, ascorbic acid, and heavy metals, such as manganese, cobalt, magnesium, or salt thereof. Examples of preservatives that can be used include phenol, benzalkonium chloride, benzyl alcohol, chlorobutanol, and methylparaben. Examples of antiseptic agents that can be used include ethanol, benzalkonium chloride, paraoxybenzoic acid, and chlorobutanol. The content of the active ingredient (the enzyme of the present invention) in the enzyme preparation is not particularly limited, provided that the target ketose can be produced. The enzyme preparation according to the present invention is generally provided in a solid state (e.g., an enzyme immobilized on a material that can immobilize the enzyme on the surface or inside of granule, powder, silica, or porous polymer) or in a liquid state.

### 4. Use of AkDAE

AkDAE can produce allulose from a fructose substrate. Other ketose can also be produced with the use of other substrates. For example, D-sorbose can be produced from a D-tagatose substrate, or D-tagatose can be produced from a D-sorbose substrate. The optimal pH of AkDAE is low and heat resistance thereof is high. Thus, allulose can be produced at low pH and high temperatures. A reducing sugar containing allulose is likely to undergo browning, and browning can be prevented in the acidic region. In order to prevent browning, accordingly, allulose is preferably produced at low pH. Since substrate solubility is increased at high temperature, the substrate can be used at high concentration, and highly concentrated allulose can be produced as a consequence. The temperature condition of the enzymatic reaction is at 30°C to 80°C, preferably 40°C to 70°C, and more preferably 45°C to 60°C, and pH condition is 4.0 to less than 7.0, preferably 4.0 to less than 6.0, more preferably 4.0 to less than 5.0, and more preferably 4.0 or 4.5 to 4.9, 4.8, 4.7, or 4.6.

When the enzymatic reaction is performed, the substrate concentration is preferably 30 to 60 (w/v)%, and the reaction duration is preferably 10 to 180 minutes, more preferably 10 to 120 minutes, and more preferably 10 to 60 minutes.

When the reaction is allowed to proceed with the use of a 30 to 60 (w/v)% fructose substrate, the conversion rate into allulose is 20% or higher, and preferably 25% or higher. Examples

The present invention is described in greater detail with reference to the examples below, although the scope of the present invention is not limited to these examples.

### [Example 1] the allulose production test using A. krungthepensis cell lysate

D-allulose-producing cells were screened from the strain library. In the 1st screening, 70 cells were selected from among approximately 20,000 cells by TLC. TLC was performed by applying a reaction solution to the TLC Silica gel 60 plates (Merck-Millipore, Massachusetts, U.S.A.), and separating fructose from allulose with the use of a developing solvent composed of butanol:acetate:water (8:1:1). As a color developer, methanol containing 0.5% (w/v) N-(1-naphthyl)ethylenediamine dihydrochloride and 5.0% (w/v) sulfinic acid was used.

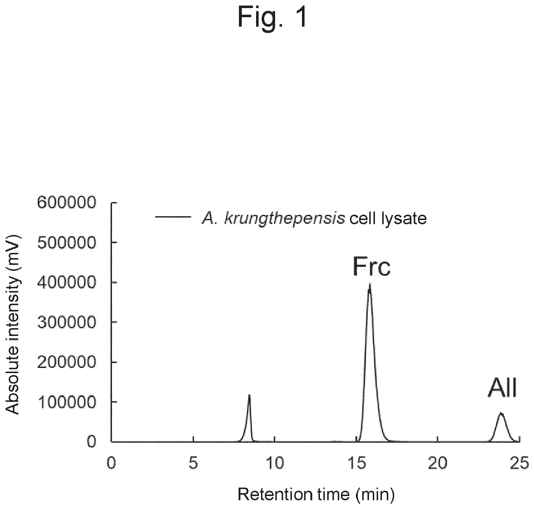

Subsequently, the cell exhibiting the highest allulose conversion rate was selected by HPLC. HPLC was performed with the use of Mili-Q as a solvent to separate fructose from allulose using MCI GEL CK08EC (300 × 8.0 mm (I.D.); Mitsubishi Chemical Corporation, Tokyo, Japan) (flow rate: 0.6 ml/min; temperature: 75°C), and isomerized sugars were detected using the evaporative light scattering detection system (Shimadzu, Kyoto, Japan). As a result, *Asaia krungthepensis* was found to exhibit the highest allulose conversion rate, and the conversion rate into allulose was 28.9% (Fig. 1).

### [Example 2] Sequence determination

Degenerate primers (Fw: 5'-ATGAACAAGCTYGGAYTSCAYGC-3' (SEQ ID NO: 1); Rv: 5'-TCAGCGSTTRTGGCGYCCYAGCGC-3' (SEQ ID NO: 2)) were designed based on the homolog sequence of the genus *Asaia.* With the use of the designed primers and the *A. krungthepensis* genome, the putative allulose-producing enzyme (AkDAE) gene was amplified by PCR. On the basis of the amplified internal sequence, subsequently, primers (Rv: 5'-ATCCATATCTGCAGTCAAGCC-3' (SEQ ID NO: 3); Fw: 5'-TCGATTTCCCGAGCATATTCC-3' (SEQ ID NO: 4)) were designed, and the upstream sequence and the downstream sequence of the putative allulose-producing enzyme gene were determined. The amino acid sequence deduced based on the AkDAE gene obtained is shown below.

MNKLGLHAFVWTAGWTPEDAAMAIRSTAELGYDLIEASTMDLKAFDVPATLRELEA
NRLGITMSFGLTADMDISSGDPERIRRGEAHLLDAISLARDVGATHVCGILYSAFQKYA
TPVTAEGVRGSIEVVRRVAEKAQASGITLGMEVVNRYESNVLNTARQAIAYVKRVEM
PNVKLHLDCYHMNIEEADSAEAIREAGDLLGYFHTGDSHRGYLGSGSVDFPSIFRALE

SIQYQGPITFESFSSAVVGQPLEGILGIWRNLWEDSRDLAAHAKAFTDVQMKSAREAL
GRQKR (SEQ ID NO: 6)

### [Example 3] Conversion rate in high-density substrate solution (heterologous expression)

The full-length AkDAE sequence was amplified by PCR and subjected to treatment with a restriction enzyme *Nco*I. The resultant was ligated to the pQE-60 vector, which had also been subjected to treatment with a restriction enzyme *Nco*I, to transform the *E.coli* JM109 cell. The recombinant *E*. *coli* cell was treated with 0.1 mM IPTG to induce AkDAE and purified with nickel-nitrilotriacetic acid agarose (QIAgen, Hilden, Germany). The allulose conversion rate of AkDAE attained when 30 to 60 (w/v)% fructose substrate solutions were used was examined. The incubation reaction was performed using a 50 mM Tris-HCl buffer (pH 7.5) at 50°C for 1 hour. As a result, the allulose conversion rates when 30 to 60 (w/v)% substrate solutions were used were 28.5%, 28.2%, 28.2%, and 28.4% (Table 1). The results demonstrate that AkDAE is the allulose-producing enzyme derived from *A. krungthepensis,* which had been cloned herein.

**[Table 1]**

| Substrate | | Allulose conversion rate (%) | | |
|---|---|---|---|---|
| | | 1 | 2 | 3 |
| Fructose | 30% | 28.7 | 28.9 | 27.8 |
| | 40% | 27.9 | 28.2 | 28.4 |
| | 50% | 28.3 | 28.4 | 28.0 |
| | 60% | 28.9 | 28.1 | 28.3 |

### [Example 4] Optimal pH (heterologous expression)

The optimal pH of AkDAE was inspected.

The buffers indicated below were used:
A 50 mM acetate buffer (pH 4.0 to 6.0), a 50 mM phosphate buffer (pH 6.0 to 7.0), a 50 mM Tris-HCl buffer (pH 7.0 to 9.0), and a 50 mM glycine-NaOH buffer (pH 9.0 to 11.0).
DAE activity was assayed by subjecting 0.2 ml of a reaction solution containing 60.0 (w/v)% fructose and 50 µM purified AkDAE to a reaction at 70°C for 1 hour. After the completion of the reaction, the resultant was subjected to boiling treatment at 100°C for 5 minutes. After the resultant was allowed to cool, fructose and allulose were detected by HPLC, and AkDAE activity was determined by designating the amount of the enzyme producing 1 mmol of allulose in a minute as 1 unit. AkDAE activity was evaluated relative to the activity at a pH of 7.0 designated 100%.

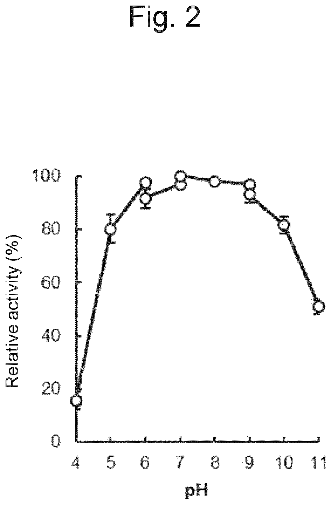

The results are shown in Fig. 2. As shown in Fig. 2, AkDAE activity at a pH of 5.0 was 80% or higher relative to the activity at the optimal pH.

### [Example 5] pH stability (heterologous expression)

pH stability of AkDAE was inspected.

The buffers indicated below were used:
50 mM acetate buffer (pH 3.0 to 6.0), 50 mM phosphate buffer (pH 6.0 to 7.0), 50 mM Tris-HCl buffer (pH 7.0 to 9.0), and 50 mM glycine-NaOH buffer (pH 9.0 to 11.0).
50 µM purified AkDAE was dissolved in buffers at relevant pH and treated at 4°C for 24 hours. After the treatment, 0.2 ml of a reaction solution containing 60.0 (w/v)% fructose and pretreated 50 µM purified AkDAE was subjected to a reaction at 70°C for 1 hour. After the completion of the reaction, the resultant was subjected to boiling treatment at 100°C for 5 minutes. After the resultant was allowed to cool, fructose and allulose were detected by HPLC, and AkDAE activity was determined by designating the amount of the enzyme producing 1 mmol of allulose in a minute as 1 unit. AkDAE activity was evaluated relative to the activity of an unreacted sample designated 100%.

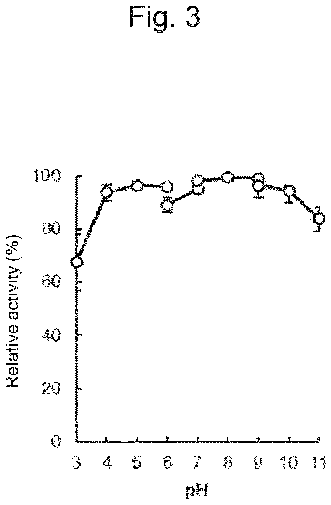

The results are shown in Fig. 3. As shown in Fig. 3, 90% or more AkDAE activity remained in a pH range of 4 to 9, and 60% or more AkDAE activity remained in a pH range of 3 to 10.

### [Example 6] Optimal temperature

Optimal temperature of AkDAE was inspected.

A reaction temperature was 30°C to 80°C.

DAE activity was assayed by subjecting 0.2 ml of a reaction solution containing a 50 mM phosphate buffer (pH 7.0), 60.0 (w/v)% fructose, and 50 µM purified AkDAE to a reaction for 1 hour. After the completion of the reaction, the resultant was subjected to boiling treatment at 100°C for 5 minutes. After the resultant was allowed to cool, fructose and allulose were detected by HPLC, and AkDAE activity was determined by designating the amount of the enzyme producing 1 mmol of allulose in a minute as 1 unit. AkDAE activity was evaluated relative to the activity at 65°C designated 100%.

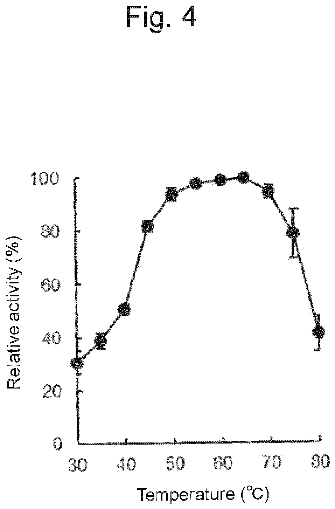

The results are shown in Fig. 4. As shown in Fig. 4, 80% or more AkDAE activity remained in a temperature range of 45°C to 70°C.

### [Example 7] Heat stability

Heat stability of AkDAE was inspected.

A 50 mM Tris-HCl buffer (pH 7.5) was used.

Pretreatment was performed at 50°C, 60°C, 70°C, and 80°C for 0, 15, 30, 45, and 60 minutes. After the pretreated 50 µM purified AkDAE was allowed to cool, 0.2 ml of a reaction solution containing 60.0 (w/v)% fructose and 50 µM purified AkDAE was subjected to a reaction at 70°C for 1 hour. After the completion of the reaction, the resultant was subjected to boiling treatment at 100°C for 5 minutes. After the resultant was allowed to cool, fructose and allulose were detected by HPLC, and AkDAE activity was determined by designating the amount of the enzyme producing 1 mmol of allulose in a minute as 1 unit. AkDAE activity was evaluated relative to the activity of an unreacted sample designated 100%.

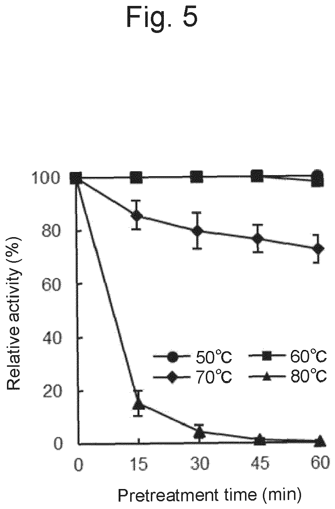

The results are shown in Fig. 5. After the treatment at 50°C or 60°C for 1 hour, the residual activity of AkDAE was 100%. The residual activity was 72% when treatment was performed at 70°C for 1 hour. The results demonstrate that AkDAE is a heat-resistant allulose-producing enzyme.

### [Example 8] Substrate specificity

Substrate specificity of AkDAE was inspected.

As substrates, 60.0 (w/v)% D-allulose (D-All), D-fructose (D-Frc), D-tagatose (D-Tag), and D-sorbose (D-Sor) were used. As a buffer, a 50 mM Tris-HCl buffer (pH 7.5) was used. A reaction solution (0.2 ml) containing a relevant substrate and 50 µM purified AkDAE was subjected to a reaction at 70°C for 1 hour. After the completion of the reaction, the resultant was subjected to boiling treatment at 100°C for 5 minutes. After the resultant was allowed to cool, the substrate and the product were detected by HPLC, and activity was determined by designating the amount of the enzyme producing 1 mmol of isomerized sugar from each substrate in a minute as 1 unit. Activity was evaluated relative to the activity attained with the use of the D-allulose substrate, which was designated 100%.

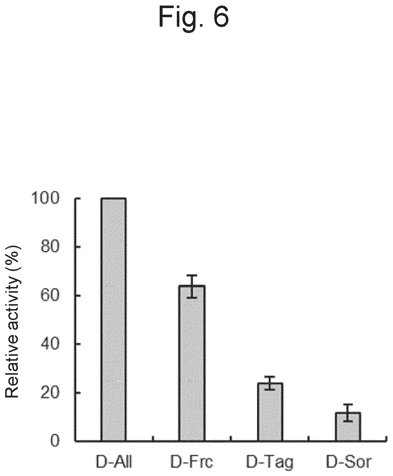

The results are shown in Fig. 6. Substrate specificity of AkDAE to D-allulose was the highest. Relative activity to D-fructose, D-tagatose, and D-sorbose was 63.6%, 23.8%, and 11.5%, respectively.

### [Example 9] Rare sugar conversion rate

The rare sugar conversion rate of AkDAE was inspected.

As substrates, 60.0 (w/v)% D-allulose (D-All), D-fructose (D-Frc), D-tagatose (D-Tag), and D-sorbose (D-Sor) were used. As a buffer, a 50 mM Tris-HCl buffer (pH 7.5) was used. A reaction solution (0.2 ml) containing a relevant substrate and 50 µM AkDAE was subjected to a reaction at 70°C for 4 hours. After the completion of the reaction, the resultant was subjected to boiling treatment at 100°C for 5 minutes. After the resultant was allowed to cool, the substrate and the product were detected by HPLC, and the conversion rate was determined on the basis of the proportion of the substrate to the product.

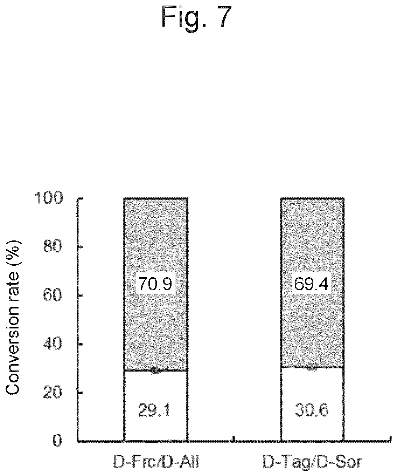

The results are shown in Fig. 7. The rare sugar conversion rates achieved by AkDAE were as follows: D-Frc:D-All of 70.9:29.1, and D-Tag:D-Sor of 69.4:30.6.

### [Example 10] Comparison under neutral pH and acidic pH conditions

The reactivity of AkDAE and that of a known allulose-producing enzymes were inspected under neutral pH and acidic pH conditions. The enzymes used were 0.5 µM AkDAE (*Asaia krungthepensis-derived* DAE), 0.5 µM AgDAE (*Arthrobacter globiformis*-derived DAE), and 2.0 µM PcDTE (*Pseudomonas cichorii*-derived DAE). Under neutral pH conditions, a 50 mM Tris-HCl buffer (pH 7.0) was used. Under acidic pH conditions, a 50 mM acetate buffer (pH 5.0) was used. The reaction was performed at 50°C for 0 to 60 minutes.

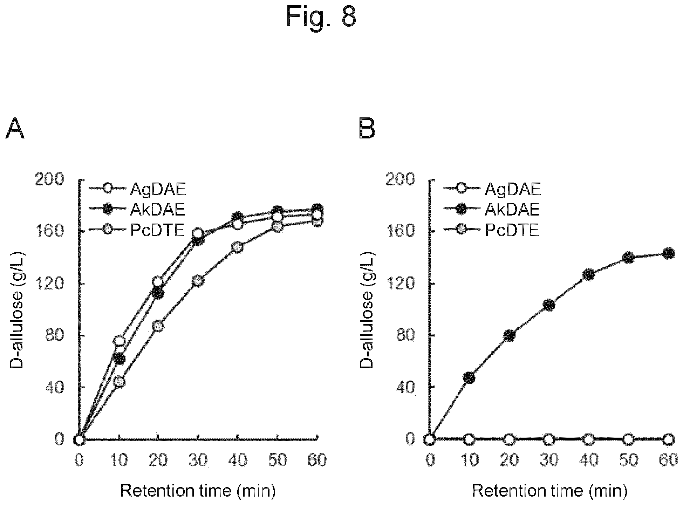

The results are shown in Fig. 8. Fig. 8A shows the results attained under neutral pH conditions, and Fig. 8B shows the results attained under acidic pH conditions. The results demonstrate that no differences were observed in allulose productivity among the 3 enzymes at a pH of 7.0 and that only AkDAE produced allulose at a pH of 5.0.

### [Example 11] Allulose production test using E. coli cells

A transformant was prepared by introducing the allulose-producing enzyme gene shown in SEQ ID NO: 1 into the *E. coli* JM109 cell. Cells were collected from the culture solution of the transformant and washed 2 times with a 50 mM Tris-HCl buffer (pH 7.5) to prepare wet cells. To a 50 mM Tris-HCl buffer (pH 7.5) containing 60.0 (w/v)% D-fructose, 50 mg of wet cells were added and mixed. The resulting mixture was treated at 50°C for 60 minutes. Cells were subjected to sampling every 10 minutes, ultrasonic fragmentation, and centrifugation at 40,000 rpm and 4°C for 10 minutes to remove cell fragments. Thereafter, the sample was analyzed by HPLC, and the amounts of allulose and fructose produced were measured.

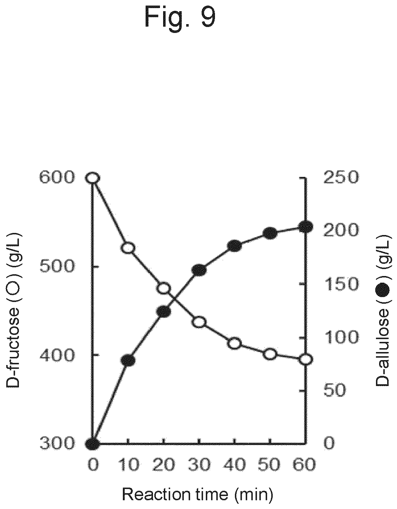

The results are shown in Fig. 9. The rare sugar conversion rate attained with the use of purified AkDAE was about 29% (Fig. 7). As a result of bioconversion with the use of *E. coli* cells, in contrast, the rare sugar conversion rate was 34%.

### [Example 12] Comparison test with analogous enzymes

The allulose-producing capacity of the allulose-producing enzymes derived from acetic acid bacteria other than *Asaia krungthepensis* was compared with that of AkDAE. The amino acid sequence identity between the allulose-producing enzymes inferred from the genomic information of acetic acid bacteria other than *Asaia krungthepensis* and AkDAE is as described below.
Sequence identity to *Gluconobacter thailandicus* (NBRC100600): 31.1%
Sequence identity to *Acetobacter indonesiensis* (NBRC16471): 26.8%
Sequence identity to *Neoasaia chiangmaiensis* (NBRC101099): 48.0%
Sequence identity to *Swaminathania salitolerans* (NBRC104436): 81.5%

Bacterial cells were inoculated into 20 ml of a medium prepared in a 100-ml triangular flask (2 (w/v)% allulose, 0.52 (w/v)% ammonium sulfate, 0.24 (w/v)% potassium dihydrogenphosphate, 0.56 (w/v)% dipotassium hydrogenphosphate, 0.03 (w/v)% magnesium sulfate, 0.1 (w/v)% yeast extract (Difco)), and shake culture was then performed at 30°C for 2 days. The culture solution was subjected to ultrasonic fragmentation to obtain a solution of cell lysate. To 0.8 ml of a 10 (w/v)% fructose solution (100 mM Tris-HCl (pH 7.5)), 0.1 ml of water and 0.1 ml of the solution of cell lysate were added, the reaction was allowed to proceed at 50°C for 48 hours, and the reaction was then terminated by treatment at 100°C for 10 minutes. The amount of allulose in the reaction solution was analyzed by HPLC.

**[Table 2]**

| | Allulose conversion rate (%) |
|---|---|
| *Gluconobacter thailandicus* | 7 |
| *Acetobacter indonesiensis* | 6 |
| *Neoasaia chiangmaiensis* | 12 |
| *Swaminathania salitolerans* | 10 |
| *Asaia krungthepensis* (AkDAE) | 18 |

The results are shown in Table 2. In comparison with allulose-producing enzymes derived from other acetic acid bacteria, AkDAE exhibited a higher allulose conversion rate.

### [Example 13] Comparison test with analogous enzymes (under acidic conditions)

The allulose-producing capacity of the allulose-producing enzymes derived from acetic acid bacteria other than *Asaia krungthepensis* was compared with that of AkDAE under acidic conditions. The allulose-producing capacity of AkDAE-producing cells was compared with that of *Neoasaia chiangmaiensis* (NBRC101099) and of *Swaminathania salitolerans* (NBRC104436).

Bacterial cells were inoculated into 20 ml of a medium prepared in a 100-ml triangular flask (2 (w/v)% allulose, 0.52 (w/v)% ammonium sulfate, 0.24 (w/v)% potassium dihydrogenphosphate, 0.56 (w/v)% dipotassium hydrogenphosphate, 0.03 (w/v)% magnesium sulfate, 0.1 (w/v)% yeast extract (Difco)), and shake culture was then performed at 30°C for 2 days. The culture solution was subjected to ultrasonic fragmentation to obtain a solution of cell lysate. To 10 µl of a 5 (w/v)% allulose solution (100 mM Tris-HCl (pH 7.5) or 100 mM acetic acid/sodium acetate (pH 5.0)), 10 µl of water and 10 µl of the solution of cell lysate were added, the reaction was allowed to proceed at 60°C for 1 hour, and the reaction was then terminated by treatment at 100°C for 10 minutes. The amount of fructose converted from allulose was quantified using the F-Kit D-glucose/D-fructose (Roche). Activity at a pH of 5.0 was compared based on the epimerase activity at a pH of 7.5, which was designated 100.

**[Table 3]**

| | Epimerase activity (%) | |
|---|---|---|
| | pH 5.0 | pH 7.5 |
| *Neoasaia chiangmaiensis* | 54 | 100 |
| *Swaminathania salitolerans* | 75 | 100 |
| *Asaia krungthepensis* (AkDAE) | 101 | 100 |

The results are shown in Table 3. In comparison with allulose-producing enzymes derived from other acetic acid bacteria, AkDAE exhibited epimerase activity under acidic conditions comparable to that under neutral conditions.

### Industrial Applicability

With the use of AkDAE, which is a novel ketose-3-epimerase according to the present invention, ketose, in particular, the rare sugar allulose, can be produced.

### Sequence Listing Free Text

### SEQ ID NOs: 1 to 4: Primers

All publications, patents, and patent applications cited herein are incorporated herein by reference in their entirety.

## Claims

1. A method for producing allulose comprising a step of allowing a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose to react with fructose under acidic conditions in which pH is less than 6.

2. The method according to Claim 1, wherein the acidic conditions in which pH is less than 6 are the conditions in which pH is less than 5.

3. The method according to Claim 1 or 2, wherein the polypeptide is allowed to react with fructose at 50°C to 70°C.

4. The method according to any one of Claims 1 to 3, wherein the polypeptide is derived from *Asaia krungthepensis.*

5. The method according to any one of Claims 1 to 4, wherein a microbial cell comprising a gene encoding a polypeptide capable of producing allulose in a cell is brought into contact with fructose.

6. An enzyme preparation comprising, as an active ingredient, a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose.

7. An enzyme preparation comprising a microorganism comprising a gene encoding a polypeptide consisting of the amino acid sequence shown in SEQ ID NO: 6 or a polypeptide consisting of a sequence having 90% or higher identity to the amino acid sequence shown in SEQ ID NO: 6 and capable of producing allulose.

8. The enzyme preparation according to Claim 6 or 7, wherein the polypeptide is derived from *Asaia krungthepensis.*
